# EUROPEAN PATENT APPLICATION

(11) **EP 2 128 254 A1**
(43) Date of publication of application: **02.12.2009**
(21) Application number: 08720613.2
(22) Date of filing: 26.03.2008
(51) Int. Cl.: C12N 15/09, A01H 1/00, A01N 63/00, A01P 21/00

(54) **TRANSPIRATION INHIBITOR**

(30) Priority: 28.03.2007 JP 2007083365
(71) Applicant: Scivax Corporation, Kanagawa 2130012 (JP)
(72) Inventor: TAKABE, Tetsuko, Nagoya-shi Aichi 464-0812 (JP); TAKABE, Teruhiro, Nagoya-shi Aichi 464-0812 (JP); UCHIDA, Akio, Nagoya-shi Aichi 468-0058 (JP)
(74) Representative: Gille Hrabal Struck Neidlein Prop Roos
(86) International application number: PCT/JP2008/000734
(87) International publication number: WO 2008/117537

(57) **Abstract**

There are provided an antidesiccant containing a nucleic acid as an active ingredient which codes for a heat shock protein originating from a salt tolerant cyanobacteria, an antidesiccant containing a nucleic acid which codes for a protein selected from a protein/enzyme relating to a photonic synthesis control, a protein/enzyme controlling the size of a plant body, a protein/enzyme promoting extension of a root, a protein/enzyme promoting growth of a plant body, and a protein/enzyme improving the stability of an RNA, or containing the antisense sequence thereof, a method of using the antidesiccant for making photonic synthesis more efficient, and a transpiration suppression method for a plant.

SEQUENCE LISTING

<110>  SCIVAX Corporation

<120>  New Antidesiccant

<130>  SP002800PCT

<150>  JP 07/83365
<151>  2007-03-28

<160>  2

<170>  PatentIn version 3.1

<210>  1
<211>  2166
<212>  DNA
<213>  Phanothece halophytica

<220>
<221>  CDS
<222>  (1)..(2166)
<223>

<400>  1
atg gga aaa gtt att ggt ata gat ctc ggc acc acc aac agt tgc ttt      48
Met Gly Lys Val Ile Gly Ile Asp Leu Gly Thr Thr Asn Ser Cys Phe
1               5                   10                  15

gct gtt tta gaa ggc ggt aaa ccc ctt gtt att gag aat gta gaa gga      96
Ala Val Leu Glu Gly Gly Lys Pro Leu Val Ile Glu Asn Val Glu Gly
            20                  25                  30

gga cgc aca acc cct agc att gtt gct ttt acc aaa gaa aag gag cgg      144
Gly Arg Thr Thr Pro Ser Ile Val Ala Phe Thr Lys Glu Lys Glu Arg
        35                  40                  45

tta gtg ggt caa ctg gcg aaa cga caa gcc gta acc aat gca gaa aat      192
Leu Val Gly Gln Leu Ala Lys Arg Gln Ala Val Thr Asn Ala Glu Asn
    50                  55                  60

acg att tat agc atc aag cgg ttt att gga cga cgc tgg gaa gat acc      240
Thr Ile Tyr Ser Ile Lys Arg Phe Ile Gly Arg Arg Trp Glu Asp Thr
65                  70                  75                  80

gag caa gag cgc aac cga gtt tct tac cac tgt gtt cct gga cga gat      288
Glu Gln Glu Arg Asn Arg Val Ser Tyr His Cys Val Pro Gly Arg Asp
                85                  90                  95

aaa acg gtg gat gtg aaa tgt tgg ggc aaa caa tac acg ccc caa gaa      336
Lys Thr Val Asp Val Lys Cys Trp Gly Lys Gln Tyr Thr Pro Gln Glu
            100                 105                 110

ctc tcc gct atg atc tta caa acc ctg aaa gcg gga gcc gaa gcc tat      384
Leu Ser Ala Met Ile Leu Gln Thr Leu Lys Ala Gly Ala Glu Ala Tyr
        115                 120                 125

ctc aac gag acg gta acc gaa gca gtg att aca gtt ccc gcc tat ttc      432
Leu Asn Glu Thr Val Thr Glu Ala Val Ile Thr Val Pro Ala Tyr Phe
    130                 135                 140

acc gat gcc caa cgt cag gca act aaa gac gca ggg acg atc gcg ggt      480
Thr Asp Ala Gln Arg Gln Ala Thr Lys Asp Ala Gly Thr Ile Ala Gly
145                 150                 155                 160

ttg aat gtg atg aga att atc aat gag ccc aca gca gca gcc ctt gcc      528
Leu Asn Val Met Arg Ile Ile Asn Glu Pro Thr Ala Ala Ala Leu Ala

                165                 170                 175

ttt ggt ctc gat aag caa gaa caa aga gag cag gtt ctg gtc tat gac 576
Phe Gly Leu Asp Lys Gln Glu Gln Arg Glu Gln Val Leu Val Tyr Asp
            180                 185                 190

tta ggc ggt ggt acg ctt gat gtt tcc atc ctg caa ctg gga gat ggg 624
Leu Gly Gly Gly Thr Leu Asp Val Ser Ile Leu Gln Leu Gly Asp Gly
        195                 200                 205

gtg ttt gaa gtg aaa gca acg gcg ggg aac aac cat ctc gga gga gat 672
Val Phe Glu Val Lys Ala Thr Ala Gly Asn Asn His Leu Gly Gly Asp
    210                 215                 220

gat ttt gat aat gtc att gtc gag tgg atg ctc tca gaa ttt caa gcg 720
Asp Phe Asp Asn Val Ile Val Glu Trp Met Leu Ser Glu Phe Gln Ala
225                 230                 235                 240

caa gaa ggc atc aat ctt aat gaa gat aaa atg gca atg caa cgt ttg 768
Gln Glu Gly Ile Asn Leu Asn Glu Asp Lys Met Ala Met Gln Arg Leu
                245                 250                 255

cgg gaa gca gca gaa cgg gcc aaa atc gaa ctt tcg acg cgc cca act 816
Arg Glu Ala Ala Glu Arg Ala Lys Ile Glu Leu Ser Thr Arg Pro Thr
            260                 265                 270

acc tcc att aat tta ccc ttt att act gct ttt agt aaa gga gga ggg 864
Thr Ser Ile Asn Leu Pro Phe Ile Thr Ala Phe Ser Lys Gly Gly Gly
        275                 280                 285

gag gtt ggt ccg aaa cat tta aag tta aac tta agc cga gcc aag ttc 912
Glu Val Gly Pro Lys His Leu Lys Leu Asn Leu Ser Arg Ala Lys Phe
    290                 295                 300

aat gaa ctc tct cgt ccc ttg gtg gaa aaa aca att gat ccc ctc aag 960
Asn Glu Leu Ser Arg Pro Leu Val Glu Lys Thr Ile Asp Pro Leu Lys
305                 310                 315                 320

caa gcc att gaa gat tct gga ctt agt gtt gac caa att gat cgt atc 1008
Gln Ala Ile Glu Asp Ser Gly Leu Ser Val Asp Gln Ile Asp Arg Ile
                325                 330                 335

tta ctg gtg ggc ggg tca act cgc att cct gag gtt cag gaa gcc ttg 1056
Leu Leu Val Gly Gly Ser Thr Arg Ile Pro Glu Val Gln Glu Ala Leu
            340                 345                 350

aaa aag ttt ttt aat ggc aaa gaa cca gat cgc tcg att aac ccc gat 1104
Lys Lys Phe Phe Asn Gly Lys Glu Pro Asp Arg Ser Ile Asn Pro Asp
        355                 360                 365

gaa gct gtt gct ttg ggt gct gcg att caa ggt ggg gtg ttg ggg caa 1152
Glu Ala Val Ala Leu Gly Ala Ala Ile Gln Gly Gly Val Leu Gly Gln
    370                 375                 380

gag caa gaa gta gaa gat tta tta ctt tta gtt gtc att ccc ctt tct 1200
Glu Gln Glu Val Glu Asp Leu Leu Leu Leu Val Val Ile Pro Leu Ser
385                 390                 395                 400

tta ggc att gaa acc ctt gga gaa gta ttt aca aaa gta atc gag cgt 1248
Leu Gly Ile Glu Thr Leu Gly Glu Val Phe Thr Lys Val Ile Glu Arg
                405                 410                 415

aat acg acc att ccc acc agt aag tca caa gtg ttt tct act gcc agt 1296
Asn Thr Thr Ile Pro Thr Ser Lys Ser Gln Val Phe Ser Thr Ala Ser
            420                 425                 430

gat gga caa acc tct gta gaa att cat gtt tta caa ggg gaa cgg gcg 1344
Asp Gly Gln Thr Ser Val Glu Ile His Val Leu Gln Gly Glu Arg Ala

435                 440                 445

atg gca aat gat aat aaa acc ctt ggt aag ttc ctg ctg aca ggg att      1392
Met Ala Asn Asp Asn Lys Thr Leu Gly Lys Phe Leu Leu Thr Gly Ile
    450                 455                 460

cct gct gct ccc cga ggc gta ccc caa att gaa gtg agc ttt gat att      1440
Pro Ala Ala Pro Arg Gly Val Pro Gln Ile Glu Val Ser Phe Asp Ile
465                 470                 475                 480

gat gtc aat ggc atc ttg aaa gta tcc gca gaa gat aaa ggc act ggg      1488
Asp Val Asn Gly Ile Leu Lys Val Ser Ala Glu Asp Lys Gly Thr Gly
                485                 490                 495

cgt gaa caa ggc att gtt att aaa gaa aca ggg ggg ctg tct caa caa      1536
Arg Glu Gln Gly Ile Val Ile Lys Glu Thr Gly Gly Leu Ser Gln Gln
            500                 505                 510

gaa atc gag cgt atg cag caa gaa gcc gaa caa tat gcg gac gaa gac      1584
Glu Ile Glu Arg Met Gln Gln Glu Ala Glu Gln Tyr Ala Asp Glu Asp
        515                 520                 525

cgc aaa cgg atg caa cgg atc gaa ctt agt aac caa gcc gat agc cta      1632
Arg Lys Arg Met Gln Arg Ile Glu Leu Ser Asn Gln Ala Asp Ser Leu
    530                 535                 540

ttc tat agc cat gaa gcc act ctc aaa gat aat caa ggc tta att cca      1680
Phe Tyr Ser His Glu Ala Thr Leu Lys Asp Asn Gln Gly Leu Ile Pro
545                 550                 555                 560

caa aaa gta aaa gcc act gcg aat aag aag aaa gaa gaa ctg atg gcg      1728
Gln Lys Val Lys Ala Thr Ala Asn Lys Lys Lys Glu Glu Leu Met Ala
                565                 570                 575

ata ctt gaa gat ccc aat gtt gaa ttg gga gtc att caa act cgc ctt      1776
Ile Leu Glu Asp Pro Asn Val Glu Leu Gly Val Ile Gln Thr Arg Leu
            580                 585                 590

gaa gac tat cga caa gct gta tta agc atg gga tca gaa gtt tat agt      1824
Glu Asp Tyr Arg Gln Ala Val Leu Ser Met Gly Ser Glu Val Tyr Ser
        595                 600                 605

caa ggc agt agc aaa tct tcc gca agg gat tat gag aca gtg gga gaa      1872
Gln Gly Ser Ser Lys Ser Ser Ala Arg Asp Tyr Glu Thr Val Gly Glu
    610                 615                 620

gaa gag atg act agg gaa act gga aat tct caa gag tcc aat gct gcc      1920
Glu Glu Met Thr Arg Glu Thr Gly Asn Ser Gln Glu Ser Asn Ala Ala
625                 630                 635                 640

ttc tcc acc caa cca gag gga aca gtc aat aat gat aac ctc gaa ctg      1968
Phe Ser Thr Gln Pro Glu Gly Thr Val Asn Asn Asp Asn Leu Glu Leu
                645                 650                 655

gaa gct gtg ttt agc acg tat aat gat cac aca caa ggt tca gat gtt      2016
Glu Ala Val Phe Ser Thr Tyr Asn Asp His Thr Gln Gly Ser Asp Val
            660                 665                 670

gct acc tcc tcc cat gat gag ata gat tta gga gat gca agt gac aat      2064
Ala Thr Ser Ser His Asp Glu Ile Asp Leu Gly Asp Ala Ser Asp Asn
        675                 680                 685

tcc cag acg aac ttc gat ctc gac gat gaa gac ttt aac ccc ttt gaa      2112
Ser Gln Thr Asn Phe Asp Leu Asp Asp Glu Asp Phe Asn Pro Phe Glu
    690                 695                 700

gac ttt gat gaa gaa gag gaa gcc tca act gat gat tat gag gcg gtg      2160
Asp Phe Asp Glu Glu Glu Glu Ala Ser Thr Asp Asp Tyr Glu Ala Val

705                 710                 715                 720

gaa taa                                                             2166
Glu

<210>  2
<211>  721
<212>  PRT
<213>  Phanothece halophytica

<400>  2

Met Gly Lys Val Ile Gly Ile Asp Leu Gly Thr Thr Asn Ser Cys Phe
1               5                   10                  15

Ala Val Leu Glu Gly Gly Lys Pro Leu Val Ile Glu Asn Val Glu Gly
            20                  25                  30

Gly Arg Thr Thr Pro Ser Ile Val Ala Phe Thr Lys Glu Lys Glu Arg
        35                  40                  45

Leu Val Gly Gln Leu Ala Lys Arg Gln Ala Val Thr Asn Ala Glu Asn
    50                  55                  60

Thr Ile Tyr Ser Ile Lys Arg Phe Ile Gly Arg Arg Trp Glu Asp Thr
65                  70                  75                  80

Glu Gln Glu Arg Asn Arg Val Ser Tyr His Cys Val Pro Gly Arg Asp
                85                  90                  95

Lys Thr Val Asp Val Lys Cys Trp Gly Lys Gln Tyr Thr Pro Gln Glu
            100                 105                 110

Leu Ser Ala Met Ile Leu Gln Thr Leu Lys Ala Gly Ala Glu Ala Tyr
        115                 120                 125

Leu Asn Glu Thr Val Thr Glu Ala Val Ile Thr Val Pro Ala Tyr Phe
    130                 135                 140

Thr Asp Ala Gln Arg Gln Ala Thr Lys Asp Ala Gly Thr Ile Ala Gly
145                 150                 155                 160

Leu Asn Val Met Arg Ile Ile Asn Glu Pro Thr Ala Ala Ala Leu Ala
                165                 170                 175

Phe Gly Leu Asp Lys Gln Glu Gln Arg Glu Gln Val Leu Val Tyr Asp
            180                 185                 190

Leu Gly Gly Gly Thr Leu Asp Val Ser Ile Leu Gln Leu Gly Asp Gly
        195                 200                 205

Val Phe Glu Val Lys Ala Thr Ala Gly Asn Asn His Leu Gly Gly Asp
    210                 215                 220

Asp Phe Asp Asn Val Ile Val Glu Trp Met Leu Ser Glu Phe Gln Ala
225                 230                 235                 240

Gln Glu Gly Ile Asn Leu Asn Glu Asp Lys Met Ala Met Gln Arg Leu
                245                 250                 255

Arg Glu Ala Ala Glu Arg Ala Lys Ile Glu Leu Ser Thr Arg Pro Thr
            260                 265                 270

Thr Ser Ile Asn Leu Pro Phe Ile Thr Ala Phe Ser Lys Gly Gly Gly
        275                 280                 285

Glu Val Gly Pro Lys His Leu Lys Leu Asn Leu Ser Arg Ala Lys Phe
    290                 295                 300

Asn Glu Leu Ser Arg Pro Leu Val Glu Lys Thr Ile Asp Pro Leu Lys
305                 310                 315                 320

Gln Ala Ile Glu Asp Ser Gly Leu Ser Val Asp Gln Ile Asp Arg Ile
                325                 330                 335

Leu Leu Val Gly Gly Ser Thr Arg Ile Pro Glu Val Gln Glu Ala Leu
            340                 345                 350

Lys Lys Phe Phe Asn Gly Lys Glu Pro Asp Arg Ser Ile Asn Pro Asp
        355                 360                 365

Glu Ala Val Ala Leu Gly Ala Ala Ile Gln Gly Gly Val Leu Gly Gln
    370                 375                 380

Glu Gln Glu Val Glu Asp Leu Leu Leu Leu Val Val Ile Pro Leu Ser
385                 390                 395                 400

Leu Gly Ile Glu Thr Leu Gly Glu Val Phe Thr Lys Val Ile Glu Arg
                405                 410                 415

Asn Thr Thr Ile Pro Thr Ser Lys Ser Gln Val Phe Ser Thr Ala Ser
            420                 425                 430

Asp Gly Gln Thr Ser Val Glu Ile His Val Leu Gln Gly Glu Arg Ala
        435                 440                 445

Met Ala Asn Asp Asn Lys Thr Leu Gly Lys Phe Leu Leu Thr Gly Ile
    450                 455                 460

Pro Ala Ala Pro Arg Gly Val Pro Gln Ile Glu Val Ser Phe Asp Ile
465                 470                 475                 480

Asp Val Asn Gly Ile Leu Lys Val Ser Ala Glu Asp Lys Gly Thr Gly
                485                 490                 495

Arg Glu Gln Gly Ile Val Ile Lys Glu Thr Gly Gly Leu Ser Gln Gln
            500                 505                 510

Glu Ile Glu Arg Met Gln Gln Glu Ala Glu Gln Tyr Ala Asp Glu Asp
        515                 520                 525

Arg Lys Arg Met Gln Arg Ile Glu Leu Ser Asn Gln Ala Asp Ser Leu
    530                 535                 540

Phe Tyr Ser His Glu Ala Thr Leu Lys Asp Asn Gln Gly Leu Ile Pro
545                 550                 555                 560

Gln Lys Val Lys Ala Thr Ala Asn Lys Lys Lys Glu Glu Leu Met Ala
                565                 570                 575

Ile Leu Glu Asp Pro Asn Val Glu Leu Gly Val Ile Gln Thr Arg Leu
            580                 585                 590

Glu Asp Tyr Arg Gln Ala Val Leu Ser Met Gly Ser Glu Val Tyr Ser
        595                 600                 605

Gln Gly Ser Ser Lys Ser Ser Ala Arg Asp Tyr Glu Thr Val Gly Glu
    610                 615                 620

Glu Glu Met Thr Arg Glu Thr Gly Asn Ser Gln Glu Ser Asn Ala Ala
625                 630                 635                 640

Phe Ser Thr Gln Pro Glu Gly Thr Val Asn Asn Asp Asn Leu Glu Leu
                645                 650                 655

Glu Ala Val Phe Ser Thr Tyr Asn Asp His Thr Gln Gly Ser Asp Val
            660                 665                 670

Ala Thr Ser Ser His Asp Glu Ile Asp Leu Gly Asp Ala Ser Asp Asn
        675                 680                 685

Ser Gln Thr Asn Phe Asp Leu Asp Asp Glu Asp Phe Asn Pro Phe Glu
    690                 695                 700

Asp Phe Asp Glu Glu Glu Glu Ala Ser Thr Asp Asp Tyr Glu Ala Val
705                 710                 715                 720

Glu

SEQUENCE LISTING

<110> SCIVAX Corporation

<120> New Antidesiccant

<130> SP002800PCT

<150> JP 07/83365
<151> 2007-03-28

<160> 2

<170> PatentIn version 3.1

<210> 1
<211> 2166
<212> DNA
<213> Phanothece halophytica

<220>
<221> CDS
<222> (1)..(2166)
<223>

<400> 1
atg gga aaa gtt att ggt ata gat ctc ggc acc acc aac agt tgc ttt 48
Met Gly Lys Val Ile Gly Ile Asp Leu Gly Thr Thr Asn Ser Cys Phe
1               5                   10                  15

gct gtt tta gaa ggc ggt aaa ccc ctt gtt att gag aat gta gaa gga 96
Ala Val Leu Glu Gly Gly Lys Pro Leu Val Ile Glu Asn Val Glu Gly
            20                  25                  30

gga cgc aca acc cct agc att gtt gct ttt acc aaa gaa aag gag cgg 144
Gly Arg Thr Thr Pro Ser Ile Val Ala Phe Thr Lys Glu Lys Glu Arg
        35                  40                  45

tta gtg ggt caa ctg gcg aaa cga caa gcc gta acc aat gca gaa aat 192
Leu Val Gly Gln Leu Ala Lys Arg Gln Ala Val Thr Asn Ala Glu Asn
    50                  55                  60

acg att tat agc atc aag cgg ttt att gga cga cgc tgg gaa gat acc 240
Thr Ile Tyr Ser Ile Lys Arg Phe Ile Gly Arg Arg Trp Glu Asp Thr
65                  70                  75                  80

gag caa gag cgc aac cga gtt tct tac cac tgt gtt cct gga cga gat 288
Glu Gln Glu Arg Asn Arg Val Ser Tyr His Cys Val Pro Gly Arg Asp
                85                  90                  95

aaa acg gtg gat gtg aaa tgt tgg ggc aaa caa tac acg ccc caa gaa 336
Lys Thr Val Asp Val Lys Cys Trp Gly Lys Gln Tyr Thr Pro Gln Glu
            100                 105                 110

ctc tcc gct atg atc tta caa acc ctg aaa gcg gga gcc gaa gcc tat 384
Leu Ser Ala Met Ile Leu Gln Thr Leu Lys Ala Gly Ala Glu Ala Tyr
        115                 120                 125

ctc aac gag acg gta acc gaa gca gtg att aca gtt ccc gcc tat ttc 432
Leu Asn Glu Thr Val Thr Glu Ala Val Ile Thr Val Pro Ala Tyr Phe
    130                 135                 140

acc gat gcc caa cgt cag gca act aaa gac gca ggg acg atc gcg ggt 480
Thr Asp Ala Gln Arg Gln Ala Thr Lys Asp Ala Gly Thr Ile Ala Gly
145                 150                 155                 160

ttg aat gtg atg aga att atc aat gag ccc aca gca gca gcc ctt gcc 528
Leu Asn Val Met Arg Ile Ile Asn Glu Pro Thr Ala Ala Ala Leu Ala
                165                 170                 175

ttt ggt ctc gat aag caa gaa caa aga gag cag gtt ctg gtc tat gac 576
Phe Gly Leu Asp Lys Gln Glu Gln Arg Glu Gln Val Leu Val Tyr Asp
            180                 185                 190

tta ggc ggt ggt acg ctt gat gtt tcc atc ctg caa ctg gga gat ggg 624
Leu Gly Gly Gly Thr Leu Asp Val Ser Ile Leu Gln Leu Gly Asp Gly
        195                 200                 205

gtg ttt gaa gtg aaa gca acg gcg ggg aac aac cat ctc gga gga gat 672
Val Phe Glu Val Lys Ala Thr Ala Gly Asn Asn His Leu Gly Gly Asp
    210                 215                 220

gat ttt gat aat gtc att gtc gag tgg atg ctc tca gaa ttt caa gcg 720
Asp Phe Asp Asn Val Ile Val Glu Trp Met Leu Ser Glu Phe Gln Ala
225                 230                 235                 240

caa gaa ggc atc aat ctt aat gaa gat aaa atg gca atg caa cgt ttg 768
Gln Glu Gly Ile Asn Leu Asn Glu Asp Lys Met Ala Met Gln Arg Leu
                245                 250                 255

cgg gaa gca gca gaa cgg gcc aaa atc gaa ctt tcg acg cgc cca act 816
Arg Glu Ala Ala Glu Arg Ala Lys Ile Glu Leu Ser Thr Arg Pro Thr
            260                 265                 270

acc tcc att aat tta ccc ttt att act gct ttt agt aaa gga gga ggg 864
Thr Ser Ile Asn Leu Pro Phe Ile Thr Ala Phe Ser Lys Gly Gly Gly
        275                 280                 285

gag gtt ggt ccg aaa cat tta aag tta aac tta agc cga gcc aag ttc 912
Glu Val Gly Pro Lys His Leu Lys Leu Asn Leu Ser Arg Ala Lys Phe
    290                 295                 300

aat gaa ctc tct cgt ccc ttg gtg gaa aaa aca att gat ccc ctc aag 960
Asn Glu Leu Ser Arg Pro Leu Val Glu Lys Thr Ile Asp Pro Leu Lys
305                 310                 315                 320

caa gcc att gaa gat tct gga ctt agt gtt gac caa att gat cgt atc 1008
Gln Ala Ile Glu Asp Ser Gly Leu Ser Val Asp Gln Ile Asp Arg Ile
                325                 330                 335

tta ctg gtg ggc ggg tca act cgc att cct gag gtt cag gaa gcc ttg 1056
Leu Leu Val Gly Gly Ser Thr Arg Ile Pro Glu Val Gln Glu Ala Leu
            340                 345                 350

aaa aag ttt ttt aat ggc aaa gaa cca gat cgc tcg att aac ccc gat 1104
Lys Lys Phe Phe Asn Gly Lys Glu Pro Asp Arg Ser Ile Asn Pro Asp
        355                 360                 365

gaa gct gtt gct ttg ggt gct gcg att caa ggt ggg gtg ttg ggg caa 1152
Glu Ala Val Ala Leu Gly Ala Ala Ile Gln Gly Gly Val Leu Gly Gln
    370                 375                 380

gag caa gaa gta gaa gat tta tta ctt tta gtt gtc att ccc ctt tct 1200
Glu Gln Glu Val Glu Asp Leu Leu Leu Leu Val Val Ile Pro Leu Ser
385                 390                 395                 400

tta ggc att gaa acc ctt gga gaa gta ttt aca aaa gta atc gag cgt 1248
Leu Gly Ile Glu Thr Leu Gly Glu Val Phe Thr Lys Val Ile Glu Arg
                405                 410                 415

aat acg acc att ccc acc agt aag tca caa gtg ttt tct act gcc agt 1296
Asn Thr Thr Ile Pro Thr Ser Lys Ser Gln Val Phe Ser Thr Ala Ser
            420                 425                 430

gat gga caa acc tct gta gaa att cat gtt tta caa ggg gaa cgg gcg 1344
Asp Gly Gln Thr Ser Val Glu Ile His Val Leu Gln Gly Glu Arg Ala
        435                 440                 445

atg gca aat gat aat aaa acc ctt ggt aag ttc ctg ctg aca ggg att 1392
Met Ala Asn Asp Asn Lys Thr Leu Gly Lys Phe Leu Leu Thr Gly Ile
    450                 455                 460

cct gct gct ccc cga ggc gta ccc caa att gaa gtg agc ttt gat att 1440
Pro Ala Ala Pro Arg Gly Val Pro Gln Ile Glu Val Ser Phe Asp Ile
465                 470                 475                 480

gat gtc aat ggc atc ttg aaa gta tcc gca gaa gat aaa ggc act ggg 1488
Asp Val Asn Gly Ile Leu Lys Val Ser Ala Glu Asp Lys Gly Thr Gly
                485                 490                 495

cgt gaa caa ggc att gtt att aaa gaa aca ggg ggg ctg tct caa caa 1536
Arg Glu Gln Gly Ile Val Ile Lys Glu Thr Gly Gly Leu Ser Gln Gln
            500                 505                 510

gaa atc gag cgt atg cag caa gaa gcc gaa caa tat gcg gac gaa gac 1584
Glu Ile Glu Arg Met Gln Gln Glu Ala Glu Gln Tyr Ala Asp Glu Asp
        515                 520                 525

cgc aaa cgg atg caa cgg atc gaa ctt agt aac caa gcc gat agc cta 1632
Arg Lys Arg Met Gln Arg Ile Glu Leu Ser Asn Gln Ala Asp Ser Leu
    530                 535                 540

ttc tat agc cat gaa gcc act ctc aaa gat aat caa ggc tta att cca 1680
Phe Tyr Ser His Glu Ala Thr Leu Lys Asp Asn Gln Gly Leu Ile Pro
545                 550                 555                 560

caa aaa gta aaa gcc act gcg aat aag aag aaa gaa gaa ctg atg gcg 1728
Gln Lys Val Lys Ala Thr Ala Asn Lys Lys Lys Glu Glu Leu Met Ala
                565                 570                 575

ata ctt gaa gat ccc aat gtt gaa ttg gga gtc att caa act cgc ctt 1776
Ile Leu Glu Asp Pro Asn Val Glu Leu Gly Val Ile Gln Thr Arg Leu
            580                 585                 590

gaa gac tat cga caa gct gta tta agc atg gga tca gaa gtt tat agt 1824
Glu Asp Tyr Arg Gln Ala Val Leu Ser Met Gly Ser Glu Val Tyr Ser
        595                 600                 605

caa ggc agt agc aaa tct tcc gca agg gat tat gag aca gtg gga gaa 1872
Gln Gly Ser Ser Lys Ser Ser Ala Arg Asp Tyr Glu Thr Val Gly Glu
    610                 615                 620

gaa gag atg act agg gaa act gga aat tct caa gag tcc aat gct gcc 1920
Glu Glu Met Thr Arg Glu Thr Gly Asn Ser Gln Glu Ser Asn Ala Ala
625                 630                 635                 640

ttc tcc acc caa cca gag gga aca gtc aat aat gat aac ctc gaa ctg 1968
Phe Ser Thr Gln Pro Glu Gly Thr Val Asn Asn Asp Asn Leu Glu Leu
                645                 650                 655

gaa gct gtg ttt agc acg tat aat gat cac aca caa ggt tca gat gtt 2016
Glu Ala Val Phe Ser Thr Tyr Asn Asp His Thr Gln Gly Ser Asp Val
            660                 665                 670

gct acc tcc tcc cat gat gag ata gat tta gga gat gca agt gac aat 2064
Ala Thr Ser Ser His Asp Glu Ile Asp Leu Gly Asp Ala Ser Asp Asn
        675                 680                 685

tcc cag acg aac ttc gat ctc gac gat gaa gac ttt aac ccc ttt gaa 2112
Ser Gln Thr Asn Phe Asp Leu Asp Asp Glu Asp Phe Asn Pro Phe Glu
    690                 695                 700

gac ttt gat gaa gaa gag gaa gcc tca act gat gat tat gag gcg gtg 2160
Asp Phe Asp Glu Glu Glu Glu Ala Ser Thr Asp Asp Tyr Glu Ala Val
705                 710                 715                 720

gaa taa 2166

Glu

<210> 2
<211> 721
<212> PRT
<213> Phanothece halophytica

<400> 2

Met Gly Lys Val Ile Gly Ile Asp Leu Gly Thr Thr Asn Ser Cys Phe
1               5                   10                  15

Ala Val Leu Glu Gly Gly Lys Pro Leu Val Ile Glu Asn Val Glu Gly
            20                  25                  30

Gly Arg Thr Thr Pro Ser Ile Val Ala Phe Thr Lys Glu Lys Glu Arg
        35                  40                  45

Leu Val Gly Gln Leu Ala Lys Arg Gln Ala Val Thr Asn Ala Glu Asn
    50                  55                  60

Thr Ile Tyr Ser Ile Lys Arg Phe Ile Gly Arg Arg Trp Glu Asp Thr
65                  70                  75                  80

Glu Gln Glu Arg Asn Arg Val Ser Tyr His Cys Val Pro Gly Arg Asp
                85                  90                  95

Lys Thr Val Asp Val Lys Cys Trp Gly Lys Gln Tyr Thr Pro Gln Glu
            100                 105                 110

Leu Ser Ala Met Ile Leu Gln Thr Leu Lys Ala Gly Ala Glu Ala Tyr
        115                 120                 125

Leu Asn Glu Thr Val Thr Glu Ala Val Ile Thr Val Pro Ala Tyr Phe
    130                 135                 140

Thr Asp Ala Gln Arg Gln Ala Thr Lys Asp Ala Gly Thr Ile Ala Gly
145                 150                 155                 160

Leu Asn Val Met Arg Ile Ile Asn Glu Pro Thr Ala Ala Ala Leu Ala
                165                 170                 175

Phe Gly Leu Asp Lys Gln Glu Gln Arg Glu Gln Val Leu Val Tyr Asp
            180                 185                 190

Leu Gly Gly Gly Thr Leu Asp Val Ser Ile Leu Gln Leu Gly Asp Gly
        195                 200                 205

Val Phe Glu Val Lys Ala Thr Ala Gly Asn Asn His Leu Gly Gly Asp
    210                 215                 220

Asp Phe Asp Asn Val Ile Val Glu Trp Met Leu Ser Glu Phe Gln Ala
225                 230                 235                 240

Gln Glu Gly Ile Asn Leu Asn Glu Asp Lys Met Ala Met Gln Arg Leu
                245                 250                 255

Arg Glu Ala Ala Glu Arg Ala Lys Ile Glu Leu Ser Thr Arg Pro Thr
            260                 265                 270

Thr Ser Ile Asn Leu Pro Phe Ile Thr Ala Phe Ser Lys Gly Gly Gly
        275                 280                 285

Glu Val Gly Pro Lys His Leu Lys Leu Asn Leu Ser Arg Ala Lys Phe
    290                 295                 300

Asn Glu Leu Ser Arg Pro Leu Val Glu Lys Thr Ile Asp Pro Leu Lys
305                 310                 315                 320

Gln Ala Ile Glu Asp Ser Gly Leu Ser Val Asp Gln Ile Asp Arg Ile
                325                 330                 335

Leu Leu Val Gly Gly Ser Thr Arg Ile Pro Glu Val Gln Glu Ala Leu
            340                 345                 350

Lys Lys Phe Phe Asn Gly Lys Glu Pro Asp Arg Ser Ile Asn Pro Asp
        355                 360                 365

Glu Ala Val Ala Leu Gly Ala Ala Ile Gln Gly Gly Val Leu Gly Gln
    370                 375                 380

Glu Gln Glu Val Glu Asp Leu Leu Leu Leu Val Val Ile Pro Leu Ser
385                 390                 395                 400

Leu Gly Ile Glu Thr Leu Gly Glu Val Phe Thr Lys Val Ile Glu Arg
                405                 410                 415

Asn Thr Thr Ile Pro Thr Ser Lys Ser Gln Val Phe Ser Thr Ala Ser
            420                 425                 430

Asp Gly Gln Thr Ser Val Glu Ile His Val Leu Gln Gly Glu Arg Ala
        435                 440                 445

Met Ala Asn Asp Asn Lys Thr Leu Gly Lys Phe Leu Leu Thr Gly Ile
    450                 455                 460

Pro Ala Ala Pro Arg Gly Val Pro Gln Ile Glu Val Ser Phe Asp Ile
465                 470                 475                 480

Asp Val Asn Gly Ile Leu Lys Val Ser Ala Glu Asp Lys Gly Thr Gly
                485                 490                 495

Arg Glu Gln Gly Ile Val Ile Lys Glu Thr Gly Gly Leu Ser Gln Gln
            500                 505                 510

Glu Ile Glu Arg Met Gln Gln Glu Ala Glu Gln Tyr Ala Asp Glu Asp
        515                 520                 525

Arg Lys Arg Met Gln Arg Ile Glu Leu Ser Asn Gln Ala Asp Ser Leu
    530                 535                 540

Phe Tyr Ser His Glu Ala Thr Leu Lys Asp Asn Gln Gly Leu Ile Pro
545                 550                 555                 560

Gln Lys Val Lys Ala Thr Ala Asn Lys Lys Lys Glu Glu Leu Met Ala
                565                 570                 575

Ile Leu Glu Asp Pro Asn Val Glu Leu Gly Val Ile Gln Thr Arg Leu
            580                 585                 590

Glu Asp Tyr Arg Gln Ala Val Leu Ser Met Gly Ser Glu Val Tyr Ser
        595                 600                 605

Gln Gly Ser Ser Lys Ser Ser Ala Arg Asp Tyr Glu Thr Val Gly Glu
    610                 615                 620

Glu Glu Met Thr Arg Glu Thr Gly Asn Ser Gln Glu Ser Asn Ala Ala
625                 630                 635                 640

Phe Ser Thr Gln Pro Glu Gly Thr Val Asn Asn Asp Asn Leu Glu Leu
                645                 650                 655

Glu Ala Val Phe Ser Thr Tyr Asn Asp His Thr Gln Gly Ser Asp Val
            660                 665                 670

Ala Thr Ser Ser His Asp Glu Ile Asp Leu Gly Asp Ala Ser Asp Asn
        675                 680                 685

Ser Gln Thr Asn Phe Asp Leu Asp Asp Glu Asp Phe Asn Pro Phe Glu
    690                 695                 700

Asp Phe Asp Glu Glu Glu Glu Ala Ser Thr Asp Asp Tyr Glu Ala Val
705                 710                 715                 720

Glu

## Description

### Technical Field

The present invention relates to an antidesiccant containing a nucleic acid, which codes for a heat shock protein, as an active ingredient, or an antidesiccant for that nucleic acid.

### Background Art

A Heat Shock Protein (hereinafter, "HSP") is a protein substance whose expression is induced in accordance with a high-temperature stress, and is known as a group of protein substances called molecular chaperon. It is known that HSPs are widely present in various organism species from a procaryote to an eucaryote, and for example, (1) small HSP having a molecular weight of about 15 kDa to about 30 kDa, (2) HSP60 protein or GroEL protein (the name "HSP60" is for a eucaryote, and the name "GroEL" is for a procaryote) having a molecular weight of about 60 kDa, and (3) HSP70 protein or DnaK protein (the name "HSP70" is for a eucaryote, and the name "DnaK" is for a procaryote) having a molecular weight of about 70kDa are known.

In regard to the function of HSPs, the relationship to the high temperature tolerance is noted, but it is still under research so far, and has not been fully resolved yet.

It becomes apparent from research examples for the function of HSPs in plants that, in a tobacco, an abnormal phenotype appears if the expression of HSP60 proteins is suppressed by the antisense method (see Non-Patent Literature 1), and in an Arabidopsis, an abnormal phonotype appears if the expression of HSP70 proteins is suppressed by the antisense method (see Non-Patent Literature 2). Furthermore, it becomes known that in an Arabidopsis, the expression amount of HSP70 proteins increases if a gene which controls the transcription of a heat shock gene is suppressed, thereby obtaining an Arabidopsis having a high temperature tolerance (see Non-Patent Literature 3). Still further, it becomes known that if a gene coding for a small HSP derived from a chestnut is introduced into a colibacillus and the foregoing protein is expressed in that colibacillus, the colibacillus obtains a high temperature tolerance (see Non-Patent Literature 4).

In regard to the relationship between environmental stresses (e.g., high salt environment, dryness, or strong light) other than the high temperature tolerance and the molecular chaperon, for example, DnaK and HSP70 are relevant to the salinity tolerance, the drought resistance, and the like (see Patent Literature 1).

Patent Literature 2 discloses that HSP70 has an important function under an environment in which no particular stress is applied to a plant body. However, nowhere in Patent Literature 2 is it disclosed or suggested that the transpiration effect of water is not promoted by HSP70 even though HSP70 has effects of suppressing any transpiration of water by a plant, and of considerably promoting photonic synthesis.
[Patent Literature 1] Japanese Patent Application Laid-Open Publication No. 2001-78603
[Non-Patent Literature 1] Plant J., 6, 425 to 432 (1994)
[Non-Patent Literature 2] Mol. Gen. Genet., 252, 11 to 19 (1996)
[Non-Patent Literature 3] Plant J., 8, 603 to 612 (1995)
[Non-Patent Literature 4] Plant Physiol., 120, 521 to 528 (1999)

### Disclosure of Invention

### Problem to be Solved by the Invention

Water is necessary for cultivating plants, and a scheme of improving the water consumption efficiency is desired particularly in the field of greenery business.

### Means for Solving the Problem

In order to solve the problem, the inventor of the present invention undertook a keen examination, and amazingly found that, in comparison with a plant not subjected to recombination, a recombination plant into which HSPs are introduced suppresses any enhancement of the transpiration action even though the photonic synthesis action is considerably promoted, and accomplished the present invention using HSPs as antidesiccant.

That is, below is the summary of the present invention.
1. An antidesiccant containing a nucleic acid as an active ingredient which codes for a heat shock protein.
2. The antidesiccant according to number 1, wherein the heat shock protein is a DnaK protein or an HSP70 protein.
3. The antidesiccant according to number 2, wherein the DnaK protein is a DnaK protein of a salt tolerant cyanobacteria.
4. An antidesiccant containing a nucleic acid as an active ingredient which codes for a protein containing following amino-acid sequences described in (a) to (d).
   (a) an amino-acid sequence having amino acid numbers 1 to 721 among amino-acid sequences described in sequence number 2.
   (b) an amino-acid sequence having amino acid numbers 116 to 721 among amino-acid sequences described in sequence number 2.
   (c) an amino-acid sequence having amino acid numbers 164 to 721 among amino-acid sequences described in sequence number 2.
   (d) an amino-acid sequence having replacement, deletion, insertion or transition of one or plural amino acids in the sequences of (a) to (c).
5. The antidesiccant according to any one of numbers 1 to 4, further containing a nucleic acid which codes for greater than or equal to one protein selected from a group of a protein/enzyme relating to a control of photonic synthesis, a protein/enzyme controlling a size of a plant body, a protein/enzyme promoting extension of a root, a protein/enzyme promoting growth of a plant body, and a protein/enzyme improving a stability of an RNA, or a nucleic acid comprised of an antisense sequence thereof.
6. The antidesiccant according to any one of numbers 1 to 5, wherein the nucleic acid is a deoxyribo nucleic acid.
7. A method of using a nucleic acid, which codes for a heat shock protein, as an antidesiccant.
8. A method of using a nucleic acid, which codes for a DnaK protein or an HSP70 protein, as an antidesiccant.
9. The method according to number 8, wherein the DnaK protein is a DnaK protein of a salt tolerant cyanobacteria.
10. A method of using a nucleic acid, which codes for a protein containing following amino-acid sequences described in (a) to (d), as an antidesiccant.
   (a) an amino-acid sequence having amino acid numbers 1 to 721 among amino-acid sequences described in sequence number 2.
   (b) an amino-acid sequence having amino acid numbers 116 to 721 among amino-acid sequences described in sequence number 2.
   (c) an amino-acid sequence having amino acid numbers 164 to 721 among amino-acid sequences described in sequence number 2.
   (d) an amino-acid sequence having replacement, deletion, insertion or transition of one or plural amino acids in the sequences of (a) to (c).
11. A method of using the antidesiccant according to any one of numbers 1 to 6 for making photonic synthesis more efficient.
12. A transpiration suppression method for a plant comprising:
   introducing a nucleic acid, which codes for a heat shock protein, into a genome of a plant cell; and
   cultivating the plant cell containing the genome into which the nucleic acid is introduced.

It is to be noted that a "plant" means a plant when living things are divided into three categories: microscopic organism; plant; and animal, and includes a plant body (i.e., whole plant" and a part thereof (e.g., flower, leaf, cane, root, seed, or tissue). A "plant cell" includes, for example, a non-differentiated plant cell or a non-differentiated plant tissue culture, or a protoplast, a callus (cell conglomeration), an adventive embryo, an adventive bud, in addition to a plant cell/tissue already differentiated.
Further, a word "breed" means cultivation, incubation and the like, and represents an action of growing a plant cell, such as breeding, differentiating, enlarging, and extending.

### Effect of the Invention

The present invention provides a new use application of HSPs as an antidesiccant.

### Brief Description of Drawings

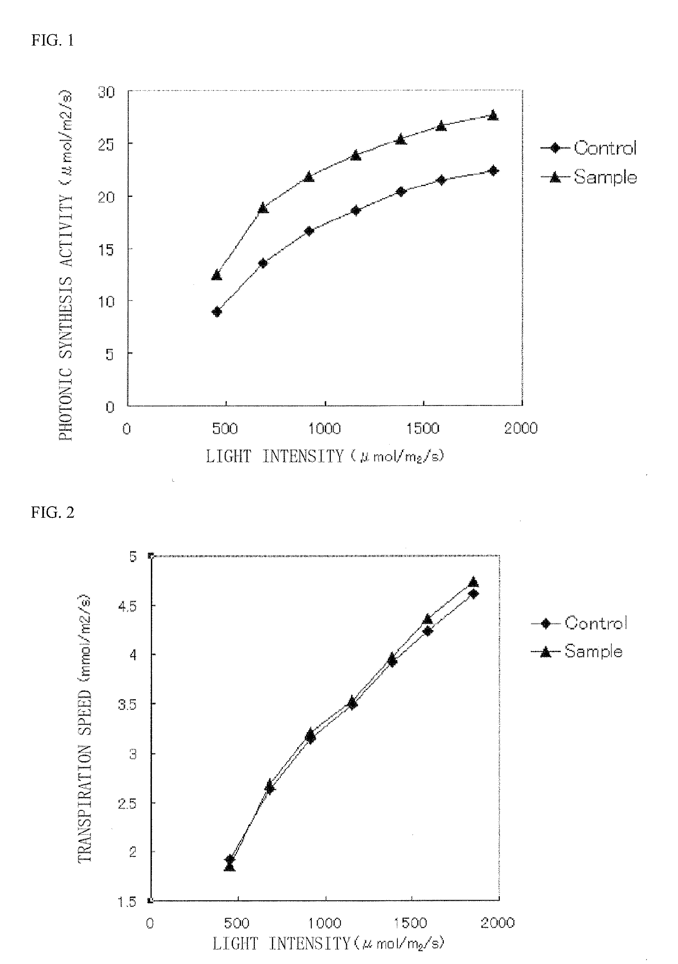

FIG. 1 is a diagram showing photonic syntheses of a rice plant on which an antidesiccant of the present invention acts and a comparative rice plant, both compared on the basis of carbon dioxide fixation amounts, where "Sample" represents the rice plant on which the antidesiccant acts, "Control" represents the comparative rice plant, and the vertical axis represents carbon dioxide fixation amounts (unit area, an amount of fixed carbon dioxide per time); and
FIG. 2 is a diagram showing transpiration speeds of a rice plant on which the antidesiccant of the present invention acts and a comparative rice plant in a comparative manner, where "Sample" represents the rice plant on which the antidesiccant of the present invention acts, "Control" represents the comparative rice plant, and the vertical axis represents transpiration speeds (unit area, a number of moles of water molecules dissipated to air per time).

### Best Mode for Carrying Out the Invention

The present invention will be explained through the best mode for carrying out the present invention.

### 1. Antidesiccant of the Present Invention

The antidesiccant of the present invention contains a nucleic acid, which codes for an HSP, as an active ingredient.
Examples of the "HSP" in the antidesiccant of the present invention are a small HSP (e.g., an HSP17.4 protein or an HSP18.2 protein or the like written in Mol. Gen. Genet., 219, 365 to 372 (1989)), an HSP60 protein (e.g., an HSP60 protein written in Plant Mol. Biol., 18, 873 to 885), a GroEl protein (e.g., a CPN10 protein written in Plant J., 10, 1119 to 1125 (1996)), an HSP70 protein (e.g., an HSC70 protein written in Plant Mol. Biol., 25, 577 to 583 (1994), an HSP70-1 protein, an HSP70-2 protein and HSP70-3 protein written in Plant Physilo., 88, 731 to 740 (1988), and a BiP protein written in Plant Cell Physiol., 37, 862 to 865 (1996)), DnaK protein, HSP 90 (an HSP81-1 protein, an HSP81-2 protein and an HSP81-3 protein written in Plant Physiol., 99, 383 to 390 (1992), an HSP81-4 protein, HSP88.1 Protein and HSP89.1 protein written in Plant Mol. Biol., 35, 955 to 961 (1997), or the like), and an HSP100 (an HSP101 1 protein written in Plant Cell, 6, 1899 to 1909 (1994)), but it is desirable that the DnaK protein and HSP70 protein should be used. It is further preferable that the DnaK protein should be used, and in particular, a DnaK protein of a salt tolerant cyanobacteria or bacteria is preferable.
An example of the HSP70 protein is an HSP70 protein of a eucaryote, and for example, an HSP70 protein of an animal. a plant, or a mycete (e.g., fungus, yeast, fungi) is exemplified.

A DnaK protein of a salt tolerant cyanobacteria is especially preferable among such proteins. The salt tolerant cyanobacteria is not limited to any particular one as long as it has a salt tolerance, but for example, a salt tolerant cyanobacteria which can be cultivated under a condition where an NaCl concentration is 0.25 mol/L to 3 mol/L, e.g., an aphanothece halophytica is exemplified. The DnaK protein of the aphanothece halophytica which is a kind of salt tolerant cyanobacteria is comprised of seven hundred and twenty one amino-acid residues, and unlike the DnaK protein of a procaryote (e.g., colibacillus or freshwater cyanobacteria) other than a salt tolerant cyanobacteria or the HSP70 protein of a eucaryote, there are extra amino-acid sequences comprised of about one hundred amino-acid residues in carboxyl group terminal (C terminal). It is known that the DnaK protein of the aphanothece halophytica has a characteristic of intervening to cause other proteins to carry out assembly correctly in a high salt concentration. The amino-acid sequence of the DnaK protein of the aphanothece halophytica is shown with sequence number 2. If a nucleic acid coding for such a DnaK protein is introduced into the genome of a plant cell, the transpiration suppression effect evaluated by a method of checking the transpiration suppression effect to be discussed later increases greater than or equal to 20 % in comparison with a plant in which the antidesiccant of the present invention is used. It is preferable that a nucleic acid used as the active ingredient of the antidesiccant of the present invention should have the transpiration suppression effect of greater than or equal to 50 %, preferably, greater than or equal to 65 %, and most preferably, greater than or equal to 80 %.

Meanwhile, regarding protein present throughout nature, in addition to polymorphism and mutation of a DNA which codes therefor, mutation, such as displacement, deletion, insertion or transition of an amino acid in an amino-acid sequence occurs due to a modification reaction in a cell of a protein after creation and during purification. Regardless of such mutation, there is known a protein which has substantially the same physiologic and biological action as that of a protein which does not have a mutation. A protein which has a slight difference in the constitution but has no large difference in the function thereof is contained in an amino-acid sequence for which a nucleic acid serving as the active ingredient of the antidesiccant of the invention codes. The same is true for a case where the foregoing mutation is artificially introduced into the amino-acid sequence of a protein, and in this case, it is possible to create various kinds of variants. For example, it is known that a polypeptide having a cysteine residue in an amino-acid sequence of human interleukin replaced by serine holds an IL-2 activity (Science, 224, 1431 (1984)). Moreover, it is known that a protein of a kind has a peptide domain which is not requisite for an activity. Examples thereof are a single peptide present in a protein secreted outside a cell, and a pro sequence which can be seen in the precursor or the like of a protease, and most domains thereof are removed after translation or when converted to an active protein. Such proteins are present with a different primary structure, but have the same function at last.

Likewise, regarding the DnaK protein and the HSP70 protein, it is needless to say that a natural DnaK protein and HSP70 protein are included, and a variant, a recombinant protein obtained through genetic engineering, and a variant protein having an amino-acid sequence in which one or more (preferably, one or several) amino acids are deleted, substituted, or added in an amino-acid sequence of a DnaK protein or an HSP70 protein are also included. Note that the expression "several" in the specification means an integer number of 7 % of the number of amino acids in an entire amino-acid sequence, preferably, an integer number of 5 %, and more preferably, an integer number of 3 %. For example, in the case of an amino-acid sequence having seven hundred and twenty one amino acids, fifty, preferably, thirty six, and more preferably, twenty one corresponds to that expression.

Meanwhile, it is known that β-1,4-galactosyltransferase contains two ATG codons in a frame (Nakagawa, K. et al. (1988), J.Biochem, 104, 165 to 168, Shaper, N. et al. (1988), J. Biol. Chem., 263, 10420 to 10428). Shaper et al discloses that β-1,4-galactosyltransferase has both forms of a longer one and a shorter one synthesized together as a result of starting translation from two portions. As explained, it is known that there are proteins having different sizes even though those proteins originate from the same gene in amino acids in nature. Likewise, for a DnaK for example, there is a possibility that a plurality of ATG codons functions as initiation codons. No matter which codon is an initiation codon, it is true that such a codon codes for the foregoing DnaK protein, and a nucleic acid starting with the second and third ATG codon can be used as the antidesiccant of the present invention. Accordingly, in addition to the amino-acid sequence having amino acid numbers 1 to 721 among amino-acid sequences of an aphanothece halophytica, an amino-acid sequence having amino acid numbers 116 to 721, and an amino-acid sequence having amino acid numbers 164 to 721 can be included.

Examples of the "nucleic acid" in the antidesiccant of the present invention are a deoxyribo nucleic acid (DNA) and a ribo nucleic acid (RNA), but it is preferable that the nucleic acid should be a DNA because the preservation stability of the antidesiccant of the present invention and the stability thereof when in use become superior. It is not limited that the nucleic acid is single strand (e.g., for a DNA, either one of the sense strand of the foregoing HSP or the antisense strand thereof) or double strand (pairing of the sense strand and the antisense strand), but double strand is preferable because the stability of the nucleic acid becomes superior. An example of the base sequence which codes for the amino-acid sequence (described in sequence number 2) of the foregoing aphanothece halophytica is a base sequence having base numbers 1 to 2166 among the base sequences described in the sequence number 1. Moreover, an example of the base sequence of a DNA which codes for an amino-acid sequence having amino acid numbers 116 to 721 in the sequence number 2 is a base sequence having base numbers 345 to 2166 among the base sequences described in the sequence number 1. Further, an example of the base sequence of a DNA which codes for an amino-acid sequence having amino acid numbers 164 to 721 in the sequence number 2, is a base sequence having base numbers 490 to 2166 among the base sequences described in the sequence number 1. Note that it is well known for the person skilled in the art that there are plural codons (base sequence corresponding to amino acid) which correspond to one amino acid. Therefore, it is easily understood by the person skilled in the art that the base sequence which codes for the amino-acid sequence described in, for example, sequence number 2 is not limited to the base sequence described in sequence number 1.

The nucleic acid (e.g., DNA) comprised of such a base sequence may have displacement, deletion, insertion or transition of one or more bases in the base sequence that constitutes the nucleic acid. Even though such mutation occurs, it hardly affects an amino-acid sequence for which the base sequence codes, and as far as the transpiration suppression effect of the antidesiccant of the present invention is maintained (it is preferable that greater than or equal to 30 % growth level through a measurement of a wetting weight should be maintained), an amino-acid sequence may have a mutation. An example of such a base sequence is a nucleic acid which hybridizes with respect to a nucleic acid (DNA or RNA) comprised of the base sequence described in sequence number 1 under a stringent condition.

Note that the "stringent condition" means a condition that sixteen hours of incubation is carried out at a temperature of 42 °C, and then successive cleaning by 1×SSPE containing 0.1 % SDS (sodium dodecyl sulfate), and 0.1×SSPE containing 0.1 % SDS is carried out at a temperature of 55 °C in the presence of, for example, 50 % formamide, 5xSSPE (water solution of pH 7.4 containing 20xSSPE: 2.97 mol/L NaCl, 0.2 mol/L NaH₂PO₄·H₂O, 0.025 mol/L EDTA (ethylenediaminetetraacetate)), 5xdenhart liquid solution (water solvent that 100xdenhart solution: 1 g of ficoll (made by Pharmacia Co., Ltd), 1 g of polyvinyl pyrolidone (PVP-360, made by Sigma Co., Ltd), and 1 g of BSA fraction V (bovine serum albumin: made by Sigma Co., Ltd) are dissolved in 50 ml of water), and 0.5 % SDS, or a condition having the same function in hybridization of a nucleic acid. It is thought that under such conditions, a DNA comprised of a base sequence that hybridizes with some DNA has at least greater than or equal to 70 % of homology with that DNA, and for example, in a DNA comprised of 2166 bases, base sequences greater than or equal to 1527 bases are in common.
Such a nucleic acid can be used as the antidesiccant (such use is called "use of the present invention").

It is preferable that the antidesiccant of the present invention should further contain a nucleic acid which codes for a protein other than an HSP. That is, because the invention is made to improve the fault in a genetically-modified plant, it is expected that the antidesiccant is used when a nucleic acid which codes for a protein other than an HSP is simultaneously introduced into a genome. Examples of such a "protein other than an HSP" are a gene like an enzyme promoting photonic synthesis (e.g., phosphoenolpyruvic acid carboxykinase (see Japanese Patent Application Laid-Open Publication No. H10-248419)), an antisense sequence which gives sterility (Bgpl (International Publication W09413809), MS2 (U.S. Patent No. 5932784), or the like), a gene which controls the size of a plant (BON1 (U.S. Patent Application Laid-Open Publication No. 2002/0194639), BAP1 (U.S. Patent Application Laid-Open Publication No. 2002/0194639), or the like), a gene related to the extension of a root (gene of proline transport factor (ProT: e.g., Plant Cell Physiol., 42 (11), 1282 to 1289 (2001)), a fructose 1.6 bisphosphate aldolase (U.S. Patent No. 6441277), a gene promoting the growth of a plant body (cyclin gene (U.S. Patent No. 6166293), and a gene for a protein which improves the stability of an RNA (mRNA) (HVD 1 gene (Japanese Unexamined Patent Application Laid-Open Publication No. 2002-34576)), and all can be used. Such a "protein other than an HSP" may be constituted in such a manner as to develop as a fusion protein with an HSP, or may be constituted in such a manner as to develop as another protein.

The foregoing nucleic acid contained in the antidesiccant of the present invention may be present as a monolithic nucleic acid, but in order to improve the stability of the nucleic acid, it is preferable that the nucleic acid should be present in a state where the nucleic acid is introduced into a recombinant vector. Examples of such a recombinant vector are binary vector pBIl21 (described in Methods Enzymol., 118, 6270640 (1986)), pCIA MBIA, and pE21-Ω-MCS, and conventional recombinant vectors can be used. It is possible for the person skilled in the art to appropriately select and use a recombinant vector in consideration of the introduction efficiency for the genome of a plant which is promoted to grow by the antidesiccant of the present invention.

In a case where a nucleic acid is introduced into a recombinant vector, it is preferable that a promoter and a terminator which function within respective target plant cells should be inserted into the upstream and downstream of the nucleic acid to be inserted, and it is preferable to insert an appropriate DNA as a selected marker which is effective when a transformant is obtained.

An example of the promoter is a 35S promoter of a cauliflower mosaic virus. An example of the terminator is a terminator originating from a nopaline synthesis enzyme (NOS). Further examples of the selected marker are a kanamycin-resistant gene (NPTII), and a hygromycin-resistant gene (HPT gene).

The antidesiccant of the present invention is available in several forms, such as liquid, powder, and paste, but other forms can be employed as long as the stability of the nucleic acid contained in the antidesiccant of the invention is not disrupted, and the antidesiccant can be used in the use example to be discussed later.

### 2. Transpiration Suppression Method of the Present Invention

The transpiration suppression method of the invention introduces a nucleic acid which codes for an HSP into the genome of a plant cell, and cultivates the plant cell containing the genome in which the nucleic acid is introduced.
The "HSP" and the "nucleic acid" of the transpiration suppression method are the same as those explained in "1. Antidesiccant of the Present Invention".

The "plant cell" of the transpiration suppression method can be a cell of either one of an angiosperm or gymnosperm, can be a cell of either one of a dicot or a monocot, and can be either one of a grass plant or a woody plant. An example of the grass plant is a cereal grain plant, a turf grass, or a vegetable, and an example of the woody plant is an evergreen broad-leaved tree, or a deciduous broad-leaved tree.

An example of the turf grass is a gramineous turf grass [e.g., eragrostis subfamily (e.g., grass group or barmuda grass group), a festuca subfamily (e.g., bent grass group, blue grass group, fescue group, or ryegrass group), or millet subfamily], a cyperaceae grass, or a chrysanthemum grass. An example of the cereal grain plant is a gramineous plant, e.g., rice plant, rye, barley, wheat, millet, common sorghum, sugarcane, corn/popcorn, or oat. Further, an example of the vegetable is a solanaceae plant (e.g., tobacco, egg plant, potato, tomato, or hot pepper), a chenopodiaceous plant (e.g., spinach, sugar beet), a papilionaceous plant (e.g., soybean, azuki bean, pisum sativum), a brassicaceous plant (e.g., oilseed rape, arugula), or a pedaliaceaeous plant (e.g., sesame).

An example of the evergreen broad-leaved tree is a eucalyptus, an acacia, or a coffee. An example of the deciduous broad-leaved tree is a poplar, a sawtooth oak, a basket willow, a Japanese white birch, or a quercus serrata.

The promoting method of the present invention is also useful for plants generally known as house plants (e.g., agavaceae, araceae, palmaceae, araliaceae, moraceae, asclepiadaceae, acanthaceae, apocynaceae, marantaceae, cupressaceae, rutaceae, kapok family, pandanaceae, musaceae, euphorbiaceae, oleaceae, commelinaceae, bromeliaceae, crassulaceae, dragon tree family, and salicaceae plants, and pteridophyte).

In such plant cells, gramineous plants, chenopodiaceous plants, leguminosae plants, brassicaceous plants, eucalyptus, acacia, and poplar are preferable, and in particular, cells of sugarcane, sugar beet, soybean, oilseed rape, sesame, eucalyptus, and poplar are more preferable, but the present invention is not limited to such plants as long as the growth of a plant is promoted.

Adopted as a method of introducing a nucleic cell into the genome of such a plant cell are a method of causing a plant tissue to get infected with an agrobacterium having a vector containing, for example, a target gene using a well-known vector (e.g., pBI121), a polyethylene glycol process method, an electroporation method of introducing the vector by performing an electric pulse process on a protoplast, or a particle gun method of superimposing the vector on a gold particle and of introducing it in a plant tissue.

A plant cell having undergone genetic transformation by the foregoing method is cultivated in an appropriate culture medium (e.g., Murashige and Skoog media, Plant Physiol., 15, 473 to 497 (1962)), and is reproduced to obtain a transformed plant. At this time, if an appropriate antibiotic agent (e.g., kanamycin) corresponding to the selected marker is added to the culture medium, it is possible to select a transformed body.

For example, in a case where a DNA which codes for a DnaK protein or an HSP70 protein is introduced into a graminaceous grass (e.g., flattened leaf or bent grass), the method of the present invention can be carried out through the following procedures.

Introduction through the foregoing polyethylene glycol process method can be carried out, for example, as follows. 1 to 100 µg/mL vector and 10⁵ to 10⁶ units/ml protoplast are suspended in a liquid of a buffer fluid containing 0.4 to 0.6 mol/L mannitol as an osmotic pressure adjusting agent. A solution of polyethylene glycol is added thereto in such a way that the final concentration becomes 10 to 30 %, the liquid is left at room temperature for 5 to 30 minutes, and then polyethylene glycol is diluted, thereby introducing a gene into the protoplast.

Introduction through the foregoing electroporation method can be carried out, for example, as follows. 1 to 100 µg/mL vector and 10⁵ to 10⁶ units/ml protoplast are suspended in a liquid of a buffer or the like containing 0.4 to 0.6 mol/L mannitol as an osmotic pressure adjusting agent. Electric pulses having an electromagnetic field of 300 to 600 V/cm and a time constant of 10 to 50 ms are applied to the liquid to apply electrical impulse, thereby introducing the gene into the protoplast.

Introduction through the foregoing particle gun method can be carried out, for example, as follows. A cell conglomeration (0.3 to 0.5 ml or so) is uniformly spread and adsorbed on a filter paper. Metal particles each having a diameter of 0.1 to 5 µm in which the vector is adsorbed using calcium chloride and spermidine are prepared, and are hit in and introduced into the cell conglomeration by a particle gun at an air pressure of 800 to 1500 PSI or a pressure when explosives explode. Tungsten or gold can be used as the metal particles.

The protoplast having undergone the gene introduction process is cultivated in a liquid or solid plant-tissue culture medium, e.g., an N6 culture medium, an R2 culture medium, a K8p culture medium, or an AA medium in which a plant cultivation adjustment material, such as an auxin like 2,4-dichlorophenoxyacetic acid or naphthalene acetic acid, or a cytokinin like kinetin is added. Cultivation is carried out in a dark place, and it is preferable that the temperature should be 20 to 30 °C. A cell conglomeration is formed from the protoplast in which the gene is introduced by cultivation.

The cell conglomeration in a dedifferentiation state after the gene introduction process is cultivated in a liquid or solid plant-tissue culture medium, e.g., an N6 culture medium, an R2 culture medium, a K8p culture medium, or an AA culture medium in which plant cultivation adjustment material, such as an auxin like 2,4-dichlorophenoxyacetic acid or naphthalene acetic acid or a cytokinin like kinetin is added. Cultivation is carried out at a dark place, and it is preferable that the temperature should be 20 to 30 °C. Further, cultivation is continued with continuously-cultivated fresh culture medium at an interval of 14 to 40 days. It is preferable that cultivation is carried out in a dark place. After 24 to 48 days from the beginning of the cultivation, an adventitious embryo or an adventive bud can be obtained.

The adventitious embryo or the adventive bud is cultivated in a plant body reproduction culture medium, such as an N6 culture medium, an R2 culture medium, an MS culture medium or an LS culture medium in which a plant cultivation adjustment material, such as an auxin like 2,4-dichlorophenoxyacetic acid or naphthalene acetic acid or a cytokinin like kinetin is added. Cultivation is continued with continuosly-cultivated fresh culture medium at an interval of 14 to 40 days. It is preferable that cultivation is carried out in a dark place. After 24 to 48 days from the beginning of the cultivation, a transformed plant body can be obtained.

The plant body obtained in this fashion shows a transpiration suppression effect when a cultivation level is compared through the following method with a plant body cultivated without the transpiration suppression method of the present invention under a normal cultivation condition.

The effect of the transpiration suppression method of the invention can be checked through, for example, the following method.
That is, a method of cultivating a negative target plant into which a vector not containing an HSP is incorporated and a plant subjected to transpiration suppression, and of measuring the water absorptions of the plants after the same cultivation period, or a method of obtaining the whole plant body or a part thereof to measure the vapor emission amount to an air, and of comparing the results can be considered.
According to the transpiration suppression method of the present invention, when the negative target and the sample are caused to do photonic synthesis under the same conditions, the photonic synthesis activity increases greater than or equal to 10 % (preferably, greater than or equal to 15 %, and most preferably, greater than or equal to 20 %), and the increment of the transpiration effect is suppressed to less than or equal to 10 % (preferably, less than or equal to 5 %, and most preferably, less than or equal to 3 %).
In regard to such measurement, accurate measurement becomes possible through the method explained in the example.

### 3. How to Use the Antidesiccant of the Present Invention

The antidesiccant of the present invention can be used by introducing it into the genome of a plant cell, and cultivating or raising a plant body containing the genome having undergone the introduction.
Introduction of the antidesiccant of the present invention into the genome of a plant cell can be simply carried out through a well-known method. Examples of such a method are the method of causing a plant tissue to get infected with an agrobacterium, the polyethylene glycol process method, the electroporation method, and the particle gun method, all of which are explained in "2. Transpiration Suppression Method of the Present Invention". All of those methods use a recombinant vector.

In a case where an HSP in which a nucleic acid to be coded is inserted into a recombinant vector is used as the antidesiccant of the present invention, a recombinant vector into which the nucleic acid is incorporated or a vector prepared together with a plant subjected to growth promotion (which can be obtained by cutting out and separating a "nucleic acid which codes for an HSP" through a well-known method, and introducing it into a recombinant vector selected in accordance with a plant subjected to growth promotion) can be used.
A plant used for the antidesiccant of the present invention is a plant described in "2.
Transpiration Suppression Method of the Present Invention".

Introduction of the recombinant vector into a plant cell can be checked using an expression system like a marker gene incorporated into a recombinant vector beforehand (e.g., kanamycin-resistant gene (NPTII), hygromycin-resistant gene (HPT gene)). For example, in a case where an antibacterial-agent-resistant gene like an NPTII is used as the marker gene, a plant cell is cultivated in a culture medium containing an antibacterial agent corresponding to the resistant gene after recombination, and a plant cell to be cultivated in the culture medium is selected, thereby obtaining a strain in which the gene is introduced in the genome.
Those skilled in the art can cultivate the selected strain under appropriate conditions.

### [Example]

The present invention will be explained in more detail through the examples.

### Measurement Example 1:

### 1. Extraction of Sugar and Starch

Extraction of a sugar was carried out through the method of Rufty et al (Rufty, T.W. and Huber, S.C. 1983, Plant Physiol. 72, 474 to 480).
That is, a sugar fractionation was extracted by reducing a leaf having a fresh weight (0.2 g) to powder with liquid nitrogen, and boiling it with 5 ml of 80 % (v/v) ethanol for one hour at a temperature of 100 °C. It was subjected to centrifuging for five minutes at 3000 x g, and a supernatant was acquired. Further, a process of adding 2.5 ml of 80 % (v/v) ethanol to the residue and suspending those, and centrifuging them for five minutes at 3000 x g to obtain a supernatant was repeated twice. The sugar fractionation extracted with the 80 % (v/v) ethanol contained sucrose, hexose, pentose, and soluble origosaccharide.
In regard to extraction of the starch fractionation, 1 ml of 0.2 mol/L potassium hydride was added to the deposit, it was processed for one hour at a temperature of 100 °C, and deacidified by adding 0.2 mL of 1 mol/L acetic acid buffer (pH 5.5). Further, those were digested for 16 hours at a temperature of 37°C with 10 ml of amino glucosidase (originating from Bacillus subtilis: made by Wako Pure Chemical Industries, Ltd, 35 U/mL in 50 mmol/L acetic acid buffer, pH 5.5). The solubilized sugar was obtained as the starch fractionation.

### 2. Quantification of Sugar

Quantification of sugar was carried out through the anthrone method (Roe, J. H. 1955, J. Biol. Chem. 212: 335 to 343). That is, 20 to 100 µl of the extraction liquid of sugar obtained in 1. was added to 1 ml of anthrone test reagent (0.05 % anthrone in 66 % H₂SO₄), and caused to react for fifteen minutes at a temperature of 100 °C. Thereafter, absorbancy of 620 nm was measured with reference to glucose.

### Measurement Example 2: Measurement of Photonic Synthesis Activity

In regard to fixation of carbon dioxide and a transpiration speed, a photo synthesis system HCM-1000 (Heinz Walz GmbH, Germany) was used under conditions where CO₂ was 350 ppm, a temperature was 25 °C, and a humidity was 60 %. The measurement was carried out in a period between 11 AM and 1 PM.

### Example 1: Production of Antidesiccant of the Present Invention

An XbaI/BamHI piece containing a DnaK gene (i.e., a DNA piece obtained by digestion with restricted enzyme XbaI and BamHI) was cut out from a plasmid pEADKl having a DnaK gene of aphanothece halophytica [Plant Mol. Biol., 35, 763 to 775 (1997)], and introduced into the XbaI/BamHI recognition site of a binary vector pBI121. Through this operation, pBI121-ADK in which the DnaK gene of aphanothece halophytica was introduced was obtained between the 35S promoter of a cauliflower mosaic virus in a binary vector and a terminator originating from nopaline synthesis enzyme. This plasmid pBI121-ADK had a kanamycin-resistant gene (NPTII) originating from the binary vector pBI121 as a selected marker.
The obtained pBI121-ADK was used as an antidesiccant 1 of the present invention for a lyophilized product.

The pBI121-ADK can be used for the antidesiccant of the present invention with wide application for a gramineous plant, such as a rice plant or a wheat, a solanaceous plant like a tobacco, a grass like a Japanese lawn grass, and other various plants (including house plants).

An antidesiccant 2 of the present invention can be obtained by linking an HVD1 gene, linked to the adherence terminal of XbaI, to the upstream of a DnaK gene before insertion into the pBI121, and freeze drying pBI121-ADKHD obtained by introduction to pB121. Moreover, through the similar method, an antidesiccant 3 of the present invention which uses a ProT gene can be obtained.

By the same token, the antidesiccant 2 of the present invention containing pBI121-SH into which a small HSP (sHSP) originating from cyanobacteria obtained from Kazsa DNA Res. Inst., is incorporated, and the antidesiccant 3 of the present invention containing pBI121-H6 into which HSP60 is incorporated can be prepared.

### Example 2: Use of the Antidesiccant of the Present Invention to Rice Plant

The antidesiccant 1 of the present invention obtained through the example 1 was held in an agrobacterium tumefaciens EHA101 strain through the electroporation method. At this time, pCAMBIA [Nucleic Acid Res., 12, 8711 to 8721 (1984)] was used as a binary vector. Subsequently, genetic transformation process was performed on a rice plant (adventive embryo callus originating from the embryo of a seed of oryza sativa L. Notohilcari) using agrobacterium tumefaciens containing the antidesiccant of the present invention. It was cultivated on a Murashige and Skoog ager medium [Plant Physiol., 15, 473 to 497 (1962)] containing 3 % sucrose and 50 µg/mL hygromycin in a biologically clean environment, and two hundred and fifty plants were obtained. Fifteen kinds of independent transfectants (F0) were selected from those plants, and the seeds thereof were collected. Among fifteen kinds of F1, four kinds of those having a large DnaK-mRNA expression amount were selected, and the seeds thereof were collected. The four kinds of transfectants (F2) basically had the same phenotype. Hereinafter, one out of those four kinds was used for analysis.
A rice plant in which only pBI121 were introduced and which was cultivated for five weeks were used for comparison (see FIG. 1).

As a result, it became apparent that the rice plant using the antidesiccant 1 of the present invention increases photonic synthesis greater than or equal to 20 % in comparison with the comparative target when the photonic synthesis activity was checked in accordance with the measurement example.
On the other hand, when the transpiration effect was measured in accordance with the measurement example, the enhancement was less than 3 % for both cases (see FIG. 2).

### Example 3: Use of the Antidesiccant of the Present Invention to Tobacco

The antidesiccant 1 of the present invention obtained through the example 1 was held in an agrobacterium tumefaciens LBA4404 strain through the electroporation method. At this time, pCAMBIA [Nucleic Acid Res., 12, 8711 to 8721 (1984)] was used as a binary vector.

Subsequently, a circular leaf piece cut out from a leaf of a tobacco (Nicotiana tabacum PetitHavaba SR1) was processed using the agrobacterium tumefaciens holding the antidesiccant 1 of the present invention. It was cultivated on a Murashige and Skoog ager medium containing 3 % sucrose and 50 µg/mL kanamycin in a biologically clean environment, and two hundred and fifty plants were obtained. Fifteen kinds of independent transfectants (F0) were selected from those plants, and the seeds thereof were collected. Among fifteen kinds of F1, four kinds of those having a large DnaK-mRNA expression amount were selected, and the seeds thereof were collected. The four kinds of transfectants (F2) basically had the same phenotype.
This tobacco and a tobacco (wild tobacco) using no antidesiccant 1 of the present invention were compared with each other, so that the transpiration suppression effect of the tobacco could be checked.

### Example 4: Use of the Antidesiccant or the Present Invention to Japanese Lawn Grass

The antidesiccant 1 of the present invention prepared through the example 1 was used for the protoplast of a Japanese lawn grass (zoysia japonica) and the Japanese lawn grass having undergone such a process was cultivated, and this grass was compared with a Japanese lawn grass (wild Japanese lawn grass) using no antidesiccant 1 of the present invention, so that the transpiration suppression effect of the Japanese lawn grass could be checked.

### Example 5: Use of the Antidesiccant of the Present Invention to Bent Grass

The protoplast of a creeping bent grass (agrostis stolonifera) was processed with the antidesiccant 2 of the present invention obtained through the example 1, and this creeping bent grass was cultivated. The growth of this creeping bent grass was compared with the growth of a creeping bent grass in which an expression vector pBI121, in which only an HVD 1 gene was incorporated, was introduced, so that the transpiration suppression effect of the antidesiccant 2 of the present invention could be checked.

### Example 6: Use of the Antidesiccant of the Present Invention to Poplar

The antidesiccant 2 of the present invention obtained through the example 1 was held in an agrobacterium tumefaciens LBA4404 strain through the electroporation method. At this time, pE2113-Ω [Nucleic Acid Res., 12, 8711 to 8721 (1984)] was used as a binary vector.

Subsequently, an embryonic axis picked up from the seedling of a poplar (Populus tremula) in a biologically clean condition was soaked with the agrobacterium tumefaciens solution holding the antidesiccant 2 of the present invention for seven minutes. Thereafter, the embryonic axis was put on an LS culture medium, and cultivated for two days in a dark place. The embryonic axis was then removed, and cultivated on a CIM medium (callus induction medium: LS culture medium containing 0.1 µmol/L of 4PPU, 50 µg/mL of kanamycin, 300 µg/ml of carbenicillin, and 0.3 % gel lyte). When a shoot was formed, the chute was cut out, transferred to an RIM medium (root induction medium: LS culture medium containing 4 µmol/L of indoleacetic acid, 50 µg/ml of kanamycin, 300 µg/ml of carbenicillin, and 0.3 % of gel lyte) and cultivated. When the root split, and a plant grew up to some extent, the plant was subjected to acclimation, transferred to a pot, and cultivated, so that plant bodies were obtained. Seven kinds of independent transfectants (F0) were selected among those plant bodies, and the seeds thereof were collected. Among five kinds of F1, four kinds of transfectants having a large DnaK-mRNA expression amount were selected. The obtained transfectants (F2) had basically the same phenotype.
This poplar was compared with a poplar (wild poplar) using no antidesiccant 2 of the present invention, so that the transpiration suppression effect of the poplar could be checked.

## Claims

1. An antidesiccant containing a nucleic acid as an active ingredient which codes for a heat shock protein.

2. The antidesiccant according to claim 1, wherein the heat shock protein is a DnaK protein or an HSP70 protein.

3. The antidesiccant according to claim 2, wherein the DnaK protein is a DnaK protein of a salt tolerant cyanobacteria.

4. An antidesiccant containing a nucleic acid as an active ingredient which codes for a protein containing following amino-acid sequences described in (a) to (d).
(a) an amino-acid sequence having amino acid numbers 1 to 721 among amino-acid sequences described in sequence number 2.
(b) an amino-acid sequence having amino acid numbers 116 to 721 among amino-acid sequences described in sequence number 2.
(c) an amino-acid sequence having amino acid numbers 164 to 721 among amino-acid sequences described in sequence number 2.
(d) an amino-acid sequence having replacement, deletion, insertion or transition of one or plural amino acids in the sequences of (a) to (c).

5. The antidesiccant according to any one of claims 1 to 4, further containing a nucleic acid which codes for greater than or equal to one protein selected from a group of a protein/enzyme relating to a control of photonic synthesis, a protein/enzyme controlling a size of a plant body, a protein/enzyme promoting extension of a root, a protein/enzyme promoting growth of a plant body, and a protein/enzyme improving a stability of an RNA, or a nucleic acid comprised of an antisense sequence thereof.

6. The antidesiccant according to any one of claims 1 to 5, wherein the nucleic acid is a deoxyribo nucleic acid.

7. A method of using a nucleic acid, which codes for a heat shock protein, as an antidesiccant.

8. A method of using a nucleic acid, which codes for a DnaK protein or an HSP70 protein, as an antidesiccant.

9. The method according to claim 8, wherein the DnaK protein is a DnaK protein of a salt tolerant cyanobacteria.

10. A method of using a nucleic acid, which codes for a protein containing following amino-acid sequences described in (a) to (d), as an antidesiccant.
(a) an amino-acid sequence having amino acid numbers 1 to 721 among amino-acid sequences described in sequence number 2.
(b) an amino-acid sequence having amino acid numbers 116 to 721 among amino-acid sequences described in sequence number 2.
(c) an amino-acid sequence having amino acid numbers 164 to 721 among amino-acid sequences described in sequence number 2.
(d) an amino-acid sequence having replacement, deletion, insertion or transition of one or plural amino acids in the sequences of (a) to (c).

11. A method of using the antidesiccant according to any one of claims 1 to 6 for making photonic synthesis more efficient.

12. A transpiration suppression method for a plant comprising:
introducing a nucleic acid, which codes for a heat shock protein, into a genome of a plant cell; and
cultivating the plant cell containing the genome into which the nucleic acid is introduced.
